# EUROPEAN PATENT SPECIFICATION

(11) **EP 4 285 945 B1**
(45) Date of publication and mention of the grant of the patent: **09.07.2025**
(21) Application number: 23174168.7
(22) Date of filing: 18.05.2023
(51) Int. Cl.: A61L 15/42, A61F 13/47, A61F 13/15

(54) **SANITARY TOWEL FOR PERSONAL USE, APPARATUS AND METHOD FOR THE MANUFACTURING THEREOF**
HYGIENETUCH FÜR DEN PERSÖNLICHEN GEBRAUCH, VORRICHTUNG UND VERFAHREN ZU SEINER HERSTELLUNG
SERVIETTE HYGIÉNIQUE À USAGE PERSONNEL, APPAREIL ET PROCÉDÉ DE FABRICATION DE CELLE-CI

(30) Priority: 30.05.2022 IT 202200011354
(43) Date of publication of application: 06.12.2023
(73) Proprietor: Corman S.p.A., 20084 Lacchiarella (IT)
(72) Inventor: Migliavacca, Emilio, Lacchiarella (MI) (IT); Mantovani, Giorgio, Milano (IT); Buono, Marco, Cesano Boscone (MI) (IT); Vitale, Adriano, Milano (IT)
(74) Representative: Rastelli, Franco

(56) References cited:
- EP-A1- 2 082 714
- EP-A2- 0 494 112

## Description

### BACKGROUND OF THE INVENTION

The present invention refers to a sanitary towel for personal use and the apparatus and method for the manufacturing thereof.

The field of the invention is about the production of sanitary towels, used for personal care, such as for example women sanitary napkins, pantyliners, incontinence pads, and so on.

In the sanitary towels of the prior art the assembly of the different materials (internal Non-Woven Fabric made of cotton in contact with the skin; intermediate layers of "spun lace" absorbing material; external sheet of waterproof material) is carried out by means of the hot melt technology, in which one starts from a spun lace already pre-formed and wound on a reel, that is manufactured in a different step separated from the sanitary towel forming step, in which the cotton fibers are compacted by high-pressure water jets.

The prior art now described has the disadvantage of compacting the sanitary towel in such a way to stiffen it, causing it to lose comfort.

Moreover, both the absorption capacity and the breathability of the absorbing intermediate layer are lowered, as the squeezing produced by the high-pressure treatment tends to close the pores of the material.

The publication EP 0 494 112 A2 discloses a sanitary towel, whose absorbent layer is formed by a double layer structure made of a cellulosic pulp fluff sheet. In particular, each layer is obtained by the retting and pulping of a starting pulp board, that is substantially a sheet of paper, up to the production of a pulp fluff web, which is a sheet of paper even softer than the starting one and that, for this reason, needs to be reinforced with an analogous additional pulp fluff web.

### SUMMARY OF THE INVENTION

The aim of the present invention is to overcome the above complained disadvantages of the prior art.

Within this aim an object of the invention is to provide a new sanitary towel which, unlike the known ones, has improved softness and flexibility.

It is a further object of the invention to design a new sanitary towel having increased comfort and breathability, together with increased absorbing capacity.

It is a further object of the invention to design a new sanitary towel, a method and an apparatus, in which the intermediate layers of absorbing material may be manufactured and formed during the sanitary towel production process.

The novelty and inventive step of the invention in particular consist in that, while in the prior art pre-formed layers of absorbing material pre-wound on a reel are used, in the present invention the layer of absorbing material is produced directly and exclusively at the time of forming the overall structure of the sanitary towel as a whole. More precisely, in the method of the invention we do not start from an absorbing material already pre-formed and wound on a reel, but from a carded web of cotton fibers, subsequently calendered and whose weight and thickness are selected as a function of the required weight and thickness for the final absorbing layer.

In particular, according to the invention, the aforementioned carded web is subjected to a hot calendering process wherein:
- upon exiting the calendering process a mat of fibres of an absorbing cotton material is formed, obtained by the overlapping of the carded layers;
- the mat thus formed is passed through the rollers of the calender, with heating between 20°C and 100°C, and a compact, porous, flexible, absorbing and breathable layer comes out.

According to a preferred embodiment of the invention, to the mat thus formed a chemical-based and/or biodegradable and compostable SAP (sodium polyacrylate) is added.

These and other objects are achieved by the sanitary towel, the method and the apparatus of claims 1, 5, and 7, respectively. Some preferred embodiments of the invention result from the remaining claims.

### BRIEF DESCRIPTION OF THE DRAWINGS

These and other characteristics and advantages of the invention will be more apparent from the following non limiting description of preferred but not exclusive embodiments of the sanitary towel, of the method and of the apparatus for the manufacturing thereof, illustrated in the following figures, in which:
Figure 1 is a side elevation view of the sanitary towel according to the invention;
Figure 2 is an exploded elevation side view of the sanitary towel according to the invention;
Figure 3 is a view along section line III-III in Figure 2 according to the invention;
Figure 4 is a top view of the apparatus according to the invention;
Figure 5 is a view of the apparatus according to the invention; and
Figure 6 schematically illustrates the operation system of the carding machine of the apparatus of Figure 4.

### DETAILED DESCRIPTION OF PREFERRED EMBODIMENTS

With particular reference to the above mentioned figures, the sanitary towel for personal use of the invention is generally indicated with the numeral 1.

The sanitary towel 1 comprises at least one top layer 2 of a non-woven fabric, preferably made of cotton, at least one bottom layer 3 of a waterproof material, and at least one core layer 4 of an absorbing material, the latter being included between the top layer and the bottom layer.

According to the invention, the core layer 4 is formed by carded and calendered absorbing cotton fibres, having a substantial alignment of the fibers in a single or in a same direction.

In a preferred embodiment of the invention, the absorbing material 4 is hot calendered and features, on at least one surface or side thereof facing the non-woven fabric layer 2, an embossing 5, suitable to give greater softness and an increased absorbing power to the calendered absorbing material.

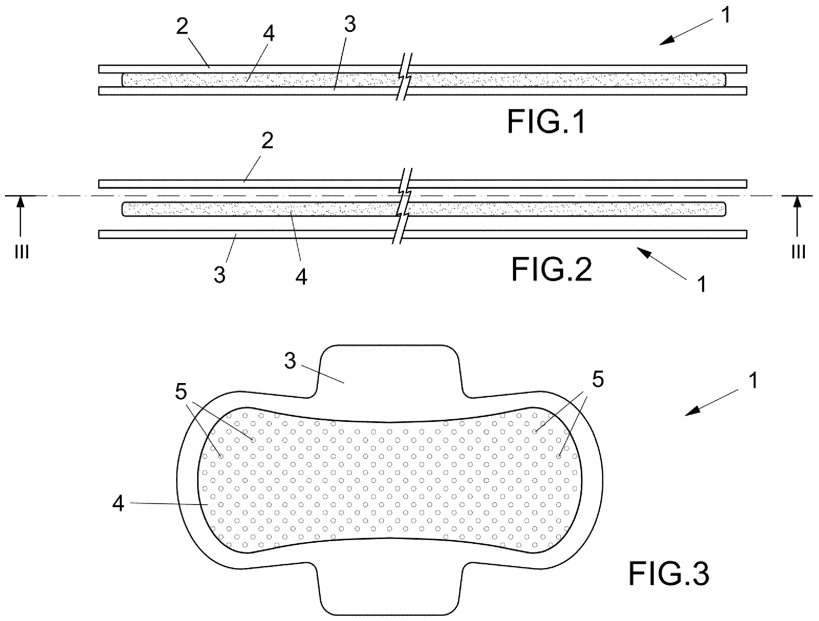

It is also an object of the present invention the method for manufacturing the sanitary towel for personal use of Figures 1 and 2, comprising at least one top layer of non-woven fabric, at least one bottom layer of waterproof material and at least one core layer of absorbing material.

The method of the invention provides in particular the manufacturing of the core layer 4 of absorbing material directly during the assembly operation of the sanitary towel 1, that is no longer from a layer of pre-formed absorbing material wound on reels.

In particular, the method consists in that the absorbing core layer 4 is manufactured carrying out the following sequence of steps.

In a first step a carding operation of cotton fibers 6 is carried out to produce at least one web 11 for the formation of the absorbing material 4 with the fibers substantially aligned in a single direction.

In particular, the cotton fibres 6 forming the web 11 have the following properties:

| | | **Units of Measure** | **Typical Analysis** |
|---|---|---|---|
| **1. Fiber Length ( by weight )** | | | |
| | **• UQL** | **cm** | **2.29 - 2.49** |
| **2. Micronaire** | | | **3.8 - 4.5** |
| **3. Absorbency ( after carding )** | | | |
| | **• Sink time** | **sec** | **2.3 - 2.5** |
| | **• Absorption capacity** | **g/g** | **26.0 - 28.0** |
| **4. Ether soluble extract** | | **%** | **0.34 - 0.40** |
| **5. Water soluble extract** | | **%** | **0.16 - 0.21** |
| **6. Sulfated ash residue** | | **%** | **0.06 - 0.11** |

In a second step a folding operation of the web 11 for the formation of a pad 13 is carried out, so as to give the required weight and thickness to the absorbing material 4 of the sanitary towel 1 of the invention.

In a third step a calendering operation of the pad thus obtained is carried out, and in a fourth step an assembly operation of the pad with the top layer of non-woven fabric and the bottom layer of waterproof material is carried out. Preferably, during the calendering operation the pad is also subjected to an embossing operation.

It is also an object of the present invention the apparatus for the manufacturing of the sanitary towel of Figures 1 and 2, in particular of the type comprising at least one top layer of non-woven fabric, at least one bottom layer of waterproof material and at least one core layer of absorbing material preferably made of cotton.

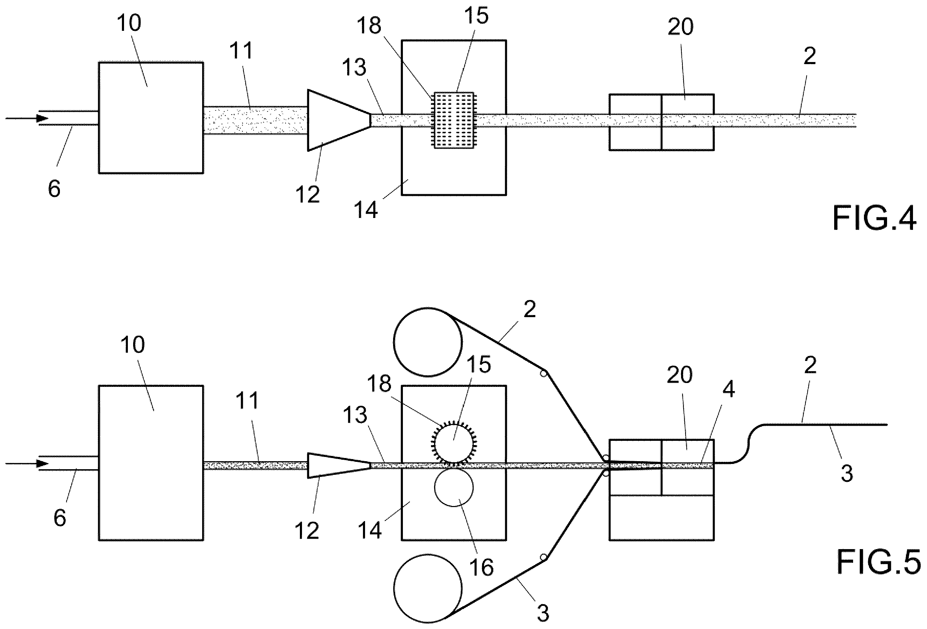

The apparatus of the invention, illustrated in Figures 4 to 6, comprises a carding machine 10 for the material forming the layer 4 of the sanitary towel 1, in particular with the purpose of aligning the fibres of this layer of material in a single or in a same direction.

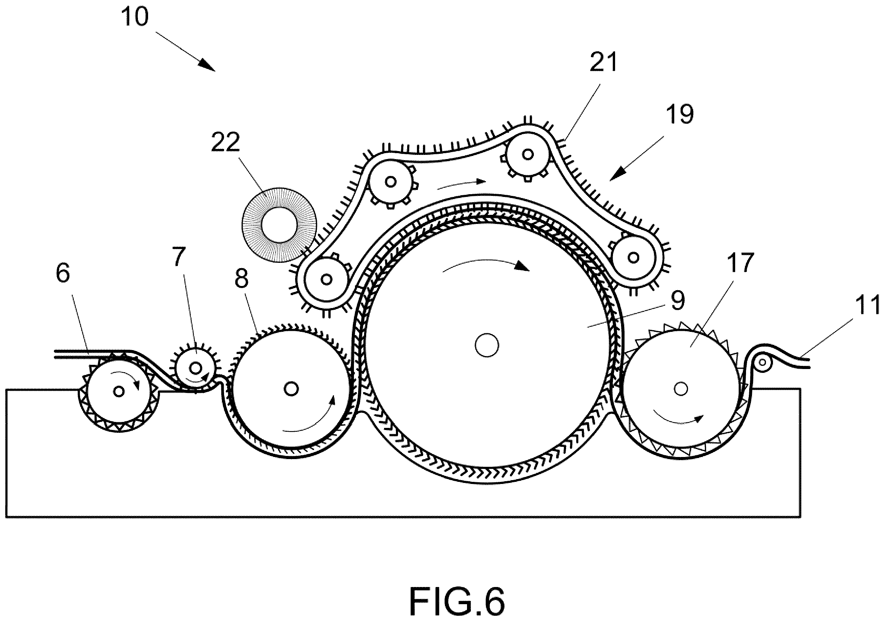

Carding is fundamental in the manufacturing cycle of the textile fibres and is intended to disentangle them in order to arrange them in a same direction. Such a process is carried out by means of the carding machine 10 in Figure 6, in which the cotton fibres 6 arrive at a conveyor cylinder 7, from here being conveyed to a licker-in cylinder 8, to a drum 9 and finally to the doffer cylinder 17, from which a web 11 of cotton fibres substantially aligned in a same direction comes out.

In the carding machine 10, defined of the "flat" type, the carding action is carried out mainly on the fibres 6 arranged between the drum 9 revolving at high speed and a plurality of plane surface flats 19 (carding torque), covered with a clothing of steel tips 21 which move, very slowly, in the same direction and which are subjected to the cleaning action of a rotating brush 22. This carding process results in the production of a cotton web 11, that is a layer of cotton wherein all the fibres are in the same direction.

According to the invention, this web 11 is processed and calendered in a calender 14, in order to give the requested absorbing properties to the sanitary towel 1. For this purpose, the web 11 may be manufactured with different weights and thicknesses, in order to increase the absorbing capacity and the thickness of the sanitary towel 1, without the need to integrate the structure with other auxiliary materials. Preferably the web 11 has a weight of 120 - 240 g/m².

The web of fibres 11 coming out of the carding machine 10 is subsequently processed in a folding machine 12, suitable to fold the web 11 in two or more layers, thus forming a pad 13 having smaller dimensions than those of the web 11 and making it suitable for entering the calender 14 for forming the layer of the absorbing material 4 having the final dimensions of the sanitary towel 1 of the invention.

The calender 14 has at least one heating roller 15 and/or at least one felt sleeve 16 for compressing the pad and producing the core layer 4 of the sanitary towel 1. Then a station 20 for assembling the pad 13 with the top layer 2 of non-woven fabric and with the bottom layer 3 of waterproof material of the sanitary towel 1 of the invention is provided.

In a preferred embodiment of the apparatus of the invention, the heating roller 15 has a plurality of etchings 18 of any pattern to produce the embossing on at least one side of the core layer.

In practice it was found that the sanitary towel for personal use and the apparatus and the method for the manufacturing thereof according to the invention are particularly advantageous for having an absorbing layer which is more flexible, softer, mechanically more resistant and with a greater absorption degree than the sanitary towels of the known type.

In the sanitary towels of the known type, in fact, the assembly is carried out with the hot melt technology, wherein one starts from a spun lace which is pre-formed and wound on a reel, in which the cotton fibres are compacted by means of high-pressure water jets with all the disadvantages deriving from this technical solution and which, as seen, are solved by the present invention.

In practice the materials used, as well as the dimensions, may be any according to the needs and the state of the art.

## Claims

1. A sanitary towel (1) for personal use comprising at least one top layer (2) of non-woven fabric, at least one bottom layer (3) of waterproof material and at least one core layer (4) of an absorbing material included between said top layer and said bottom layer, **characterized in that** said absorbing material forming said core layer (4) consists of a calendered absorbing material made of cotton, whose fibres (6) are aligned in a same direction.

2. A sanitary towel according to claim 1, **characterized in that** said absorbing material (4) is hot calendered and has an embossing (5) on at least one surface thereof facing said layer of non-woven fabric (2).

3. A sanitary towel according to claim 2, **characterized in that** it comprises a top layer (2) made of cotton and/or plastic material, at least one bottom layer (3) of waterproof material and at least one core layer (4) of an absorbing material coming from a carding machine (10).

4. A sanitary towel according to claim 3, **characterized in that** said at least one core layer (4) of an absorbing material coming from the carding machine further comprises chemical-based and/or biodegradable and compostable sodium polyacrylate.

5. A method for the manufacturing of the sanitary towel according to one or more of the preceding claims, **characterized in that** said at least one core layer (4) of absorbing material is manufactured carrying out the following steps:
- a first step, wherein at least one carding operation of cotton fibres (6) is carried out in order to produce at least one web (11) of said absorbing material, whose fibres (6) are arranged aligned in a single direction;
- a second step, wherein a folding operation of said web (11) for the formation of a pad (13) is carried out;
- a third step, wherein a calendering operation of said pad (13) is carried out; and
- at least a fourth step, wherein an operation of assembling said pad (13) with said top layer (2) of non-woven fabric and with said bottom layer (3) of waterproof material is carried out;
wherein said core layer (4) is manufactured and formed at the same time of forming the overall structure of said sanitary towel as a whole.

6. A method according to claim 5, **characterized in that**, during said calendering operation, said pad (13) is subjected to an embossing operation.

7. An apparatus for carrying out the method according to claims 5 or 6, **characterized in that** it comprises a carding machine (10) for cotton fibres (6) for producing a web (11) having fibres aligned in a same direction, a folding machine (12) suitable for folding said web (11) in the shape of a pad (13), a calender (14) being moreover provided in order to calender said pad.

8. An apparatus according to claim 7, **characterized in that** said carding machine (10) is of the "flat" type, wherein a revolving drum (9) and a plurality of plane surface flats (19) (carding torque), covered by a clothing of steel tips (21) for the processing of the aforementioned fibres (6) on the surface of said drum (9) are provided.

9. An apparatus according to claim 8, **characterized in that** said calender (14) has at least one heating roller (15) and/or at least one felt sleeve (16) for compressing said pad (13) and producing said core layer (4) of absorbing material.

10. An apparatus according to any of claims 7 to 9, **characterized in that** said heating roller (15) has a plurality of etchings (18) having patterns to produce an embossing (5) on at least one side of said core layer (4) of the aforementioned sanitary towel (1).

## Patentansprüche

1. Ein Hygienetuch (1) für den persönlichen Gebrauch, das mindestens eine Oberschicht (2) aus Vliesstoff, mindestens eine Unterschicht (3) aus wasserdichtem Material und mindestens eine zwischen der genannten Ober- und Unterschicht eingeschlossene Kernschicht (4) aus einem saugfähigen Material umfasst, **dadurch gekennzeichnet, dass** das genannte saugfähige Material, das die Kernschicht (4) bildet, aus einem kalandrierten saugfähigen Material aus Baumwolle besteht, dessen Fasern (6) in die gleiche Richtung ausgerichtet sind.

2. Ein Hygienetuch nach Anspruch 1, **dadurch gekennzeichnet, dass** das genannte saugfähige Material (4) heiß kalandriert ist und auf mindestens einer der Vliesstoffschicht (2) zugewandten Oberfläche eine Prägung (5) aufweist.

3. Ein Hygienetuch nach Anspruch 2, **dadurch gekennzeichnet, dass** es eine Oberschicht (2) aus Baumwolle und/oder Kunststoff, mindestens eine Unterschicht (3) aus wasserdichtem Material und mindestens eine Kernschicht (4) aus einem saugfähigen Material, das mit einer Kardiermaschine (10) hergestellt wird, umfasst.

4. Ein Hygienetuch nach Anspruch 3, **dadurch gekennzeichnet, dass** die genannte mindestens eine Kernschicht (4) aus einem aus der Kardiermaschine stammenden, saugfähigen Material zudem auf chemischer Basis hergestelltes und/oder biologisch abbaubares und kompostierbares Natriumpolyacrylat umfasst.

5. Ein Verfahren zur Herstellung des Hygienetuchs nach einem oder mehreren der vorhergehenden Ansprüche, **dadurch gekennzeichnet, dass** die genannte mindestens eine Kernschicht (4) aus saugfähigem Material unter Ausführung der folgenden Schritte hergestellt wird:
- ein erster Schritt, bei dem mindestens ein Vorgang zum Kardieren der Baumwollfasern (6) ausgeführt wird, um mindestens eine Bahn (11) des genannten saugfähigen Materials herzustellen, deren Fasern (6) in einer einzigen Richtung ausgerichtet angeordnet sind;
- ein zweiter Schritt, bei dem ein Faltvorgang der genannten Bahn (11) ausgeführt wird, um ein Kissen (13) zu bilden;
- ein dritter Schritt, bei dem ein Vorgang zum Kalandrieren des Kissens (13) ausgeführt wird; und
- mindestens ein vierter Schritt, bei dem ein Vorgang zum Verbinden des Kissens (13) mit der Oberschicht (2) aus Vliesstoff und der Unterschicht (3) aus wasserdichtem Material ausgeführt wird;
wobei die genannte Kernschicht (4) gleichzeitig mit der Bildung der Gesamtstruktur des Hygienetuchs hergestellt und geformt wird.

6. Ein Verfahren nach Anspruch 5, **dadurch gekennzeichnet, dass** das Kissen (13) während des Kalandriervorgangs einem Prägevorgang unterzogen wird.

7. Eine Vorrichtung zum Ausführen des Verfahrens nach Anspruch 5 oder 6, **dadurch gekennzeichnet, dass** sie eine Kardiermaschine (10) für Baumwollfasern (6) zum Erzeugen einer Bahn (11) mit in die gleiche Richtung ausgerichteten Fasern sowie eine Faltmaschine (12) zum Falten der genannten Bahn (11) in die Form eines Kissens (13) und darüber hinaus einen Kalander (14) zum Kalandrieren des Kissens umfasst.

8. Eine Vorrichtung gemäß Anspruch 7, **dadurch gekennzeichnet, dass** die Kardiermaschine (10) von "flacher Bauweise" ist, wobei eine Drehwalze (9) und eine Vielzahl von flachen Elementen (19) mit ebener Oberfläche (Kardierdrehmoment), die mit einer Garnitur aus Stahlzähnen (21) besetzt sind, zur Verarbeitung der zuvor erwähnten Fasern (6) auf der Oberfläche der Walze (9) vorgesehen sind.

9. Eine Vorrichtung nach Anspruch 8, **dadurch gekennzeichnet, dass** der genannten Kalander (14) mindestens eine Heizwalze (15) und/oder mindestens eine Filzhülse (16) zum Komprimieren des genannten Kissens (13) und Herstellen der Kernschicht (4) aus saugfähigem Material aufweist.

10. Eine Vorrichtung nach einem der Ansprüche 7 bis 9, **dadurch gekennzeichnet, dass** die genannte Heizwalze (15) eine Vielzahl von Ätzungen (18) mit Mustern aufweist, um auf mindestens einer Seite der genannten Kernschicht (4) des zuvor erwähnten Hygienetuchs (1) eine Prägung (5) zu erzeugen.

## Revendications

1. Serviette hygiénique (1) à usage personnel comprenant au moins une couche supérieure (2) de tissu non tissé, au moins une couche inférieure (3) en matériau imperméable et au moins une couche centrale (4) en matériau absorbant incluse entre ladite couche supérieure et ladite couche inférieure, **caractérisée en ce que** ledit matériau absorbant formant ladite couche centrale (4) est constitué d'un matériau absorbant calandré en coton, dont les fibres (6) sont alignées dans une même direction.

2. Serviette hygiénique selon la revendication 1, **caractérisée en ce que** ledit matériau absorbant (4) est calandré à chaud et présente un gaufrage (5) sur au moins une surface de celui-ci faisant face à ladite couche de tissu non tissé (2).

3. Serviette hygiénique selon la revendication 2, **caractérisée en ce qu'**elle comprend une couche supérieure (2) réalisée en coton et/ou en matière plastique, au moins une couche inférieure (3) en matériau imperméable et au moins une couche centrale (4) constituée d'un matériau absorbant provenant d'une machine de cardage (10).

4. Serviette hygiénique selon la revendication 3, **caractérisée en ce que** ladite au moins une couche centrale (4) constituée d'un matériau absorbant provenant de la machine de cardage comprend en outre du polyacrylate de sodium à base de produits chimiques et/ou biodégradables et compostables.

5. Procédé de fabrication de la serviette hygiénique selon l'une ou plusieurs des revendications précédentes, **caractérisé en ce que** ladite au moins une couche centrale (4) constituée d'un matériau absorbant est fabriquée en effectuant les étapes suivantes :
- une première étape, dans laquelle au moins une opération de cardage des fibres de coton (6) est effectuée afin de produire au moins une bande (11) dudit matériau absorbant, dont les fibres (6) sont disposées de manière à être alignées dans une seule direction ;
- une seconde étape, dans laquelle une opération de pliage de ladite bande (11) pour la formation d'un tampon (13) est effectuée ;
- une troisième étape, dans laquelle une opération de calandrage dudit tampon (13) est effectuée ; et
- au moins une quatrième étape, dans laquelle une opération d'assemblage dudit tampon (13) avec ladite couche supérieure (2) de tissu non tissé et avec ladite couche inférieure (3) en matériau imperméable est effectuée ;
dans lequel ladite couche centrale (4) est fabriquée et formée en même temps que la formation de la structure globale de ladite serviette hygiénique dans son ensemble.

6. Procédé selon la revendication 5, **caractérisé en ce que**, pendant ladite opération de calandrage, ledit tampon (13) est soumis à une opération de gaufrage.

7. Appareil pour mettre en œuvre le procédé selon les revendications 5 ou 6, **caractérisé en ce qu'**il comprend une machine de cardage (10) pour des fibres de coton (6) afin de produire une bande (11) ayant des fibres alignées dans une même direction, une machine à plier (12) apte à plier ladite bande (11) en forme de tampon (13), une calandre (14) étant en outre fournie pour calandrer ledit tampon.

8. Appareil selon la revendication 7, **caractérisé en ce que** ladite machine de cardage (10) est du type « plat », dans lequel un tambour rotatif (9) et une pluralité de plaques à surface plane (19) (couple de cardage), recouvertes par un revêtement de pointes en acier (21) pour le traitement des fibres (6) susmentionnées, sont fournis sur la surface dudit tambour (9).

9. Appareil selon la revendication 8, **caractérisé en ce que** ladite calandre (14) présente au moins un rouleau chauffant (15) et/ou au moins un manchon en feutre (16) pour la compression dudit tampon (13) et la production de ladite couche centrale (4) constituée de matériau absorbant.

10. Appareil selon l'une quelconque des revendications 7 à 9, **caractérisé en ce que** ledit rouleau chauffant (15) a une pluralité de gravures (18) ayant des motifs pour produire un gaufrage (5) sur au moins un côté de ladite couche centrale (4) de la serviette hygiénique (1) susmentionnée.
